# EUROPEAN PATENT APPLICATION

(11) **EP 4 603 839 A1**
(43) Date of publication of application: **20.08.2025**
(21) Application number: 23877372.5
(22) Date of filing: 13.10.2023
(51) Int. Cl.: G01N 33/86, A61K 31/198, A61K 31/785, A61K 33/00, A61K 33/06, A61K 33/26, A61K 33/34, A61P 7/04, A61P 43/00, C08F 20/36

(54) **BLOOD COAGULATION TIME SHORTENING AGENT FOR BLOOD SPECIMEN DEFICIENT IN COAGULATION FACTOR XII**

(30) Priority: 14.10.2022 JP 2022165827
(71) Applicant: Sekisui Medical Co., Ltd., Tokyo 103-0027 (JP)
(72) Inventor: YAMAMOTO, Mitsuaki, Tokyo 103-0027 (JP); BANBA, Yoshimasa, Tokyo 103-0027 (JP); HORIUCHI, Ryousuke, Tokyo 103-0027 (JP); MACHIDA, Satoshi, Tokyo 103-0027 (JP)
(74) Representative: Wächtershäuser & Hartz Patentanwaltspartnerschaft mbB
(86) International application number: PCT/JP2023/037274
(87) International publication number: WO 2024/080371

(57) **Abstract**

A blood coagulation time shortening agent for a blood specimen deficient in coagulation factor XII, in activated partial thromboplastin time measurement includes, as an active ingredient, a polymer having 2-methacryloyloxyethyl phosphorylcholine as a constitutional unit.

## Description

### Technical Field

The present invention relates to a blood coagulation time shortening agent for a blood specimen deficient in coagulation factor XII in activated partial thromboplastin time (APTT) measurement.

### Background Art

A blood coagulation test is a test for diagnosing the blood coagulation ability of a patient by adding a predetermined reagent to a blood specimen of the patient and measuring the blood coagulation time or the like. Typical examples of the blood coagulation time are prothrombin time (PT), activated partial thromboplastin time (APTT), thrombin time, and the like. An abnormality in blood coagulation ability causes prolongation of coagulation time. Examples of the cause of prolongation of coagulation time include a coagulation inhibitor (such as heparin), a decrease in coagulation-related factor, innate deficiency of a blood coagulation factor (such as hemophilia), and an autoantibody that inhibits coagulation reaction (such as Lupus anticoagulant (LA) and an anticoagulation factor antibody).

In the blood coagulation test, the reagent is added to a blood specimen, the subsequent blood coagulation reaction is measured, and the blood coagulation time is measured from the coagulation reaction. When prolongation of the coagulation time is observed, it is judged that there is an abnormality in the blood coagulation ability. The reagent for a blood coagulation test includes an ingredient for promoting blood coagulation under a certain condition. For example, an APTT measurement reagent includes a coagulation factor activator such as ellagic acid, a phospholipid, a buffer, and so on. There are various types in commercially available APTT measurement reagents, and the sensitivity to each coagulation abnormality is different among these reagents.

Patent Literature 1 discloses an APTT measurement reagent containing manganese chloride as an activity enhancing agent and glycine as a suspension reinforcing means. Patent Literature 2 states that a mixture of aluminum chloride, ferric chloride, or manganese chloride and ellagic acid activates coagulation of specimens, although ferric chloride causes precipitate of ellagic acid. Patent Literature 3 states that glycylglycine or glycylglycylglycine is used as a blood coagulation factor stabilizer in APTT measurement, although the coagulation time is prolongated when they are added in high concentrations. Patent Literature 4 describes states that as diluent for a blood coagulation ability measurement specimen, a Good's buffer such as HEPES, PIPES, POPSO, ACES, TAPSO, EPPS, Tricine, or CHES can be used. Patent Literature 5 states that an APTT measurement reagent including predetermined concentrations of phosphatidylcholine, phosphatidylserine, and phosphatidylethanolamine improves the sensitivity to LA and has appropriate sensitivity to heparin. Patent Literature 6 states that the quality of an APTT measurement reagent is stabilized by a polymer containing a (meth)acrylic acid monomer unit having a phosphorylserine residue.

### Citation List

### Patent Literature

Patent Literature 1: JP-A-59-91899
Patent Literature 2: JP-A-2002-156379
Patent Literature 3: JP-B-6-50999
Patent Literature 4: JP-A-2013-145238
Patent Literature 5: JP-A-2020-56578
Patent Literature 6: JP-A-2021-181929

### Summary of Invention

### Technical Problem

In a blood coagulation test, it is desirable to be able to accurately detect specimens having coagulation abnormalities (such as coagulation factor deficiency, LA positive, and coagulation inhibitor positive). For example, it is desirable that the blood coagulation times of blood specimens having coagulation abnormalities are regulated such that the blood specimens can be precisely detected based on the blood coagulation times.

### Solution to Problem

The present invention provides a blood coagulation time shortening agent for a specimen deficient in coagulation factor XII (FXII) in APTT measurement, and an APTT measurement method using it, and a method for shortening the blood coagulation time of an FXII-deficient specimen in APTT measurement.

As embodiments of the present invention, the followings are provided:
[1] A blood coagulation time shortening agent for a blood specimen deficient in coagulation factor XII in activated partial thromboplastin time measurement, comprising, as an active ingredient, a polymer having 2-methacryloyloxyethyl phosphorylcholine as a constitutional unit;
[2] The shortening agent according to [1], wherein the polymer is a copolymer of 2-methacryloyloxyethyl phosphorylcholine and another monomer having a hydrophobic group, an anionic group, or a cationic group;
[3] The regulator according to [2], wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C;
[4] The shortening agent according to any one of [1] to [3], further comprising at least one selected from the group consisting of a metal salt compound and an amino acid as an active ingredient;
[5] The shortening agent according to [4], wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof;
[6] The shortening agent according to [4] or [5], wherein the amino acid is at least one selected from the group consisting of α-alanine, arginine, asparagine, aspartic acid, glutamic acid, glycine, glycylglycine, methionine, phenylalanine, proline, serine, threonine, valine, and tricine;
[7] An activated partial thromboplastin time measurement reagent comprising the blood coagulation time shortening agent according to any one of [1] to [6];
[8] An activated partial thromboplastin time measurement method comprising measuring a blood coagulation time of a sample solution containing a blood specimen and the blood coagulation time shortening agent according to any one of [1] to [6];
[9] A method for shortening blood coagulation time of a blood specimen deficient in coagulation factor XII in activated partial thromboplastin time measurement, comprising measuring blood coagulation time of a sample solution containing the blood specimen and the blood coagulation time shortening agent according to any one of [1] to [6];
[10] Use of a polymer having 2-methacryloyloxyethyl phosphorylcholine as a constitutional unit for shortening blood coagulation time of a blood specimen deficient in coagulation factor XII in activated partial thromboplastin time measurement;
[11] The use according to [10], wherein the polymer is a copolymer of 2-methacryloyloxyethyl phosphorylcholine and another monomer having a hydrophobic group, an anionic group, or a cationic group;
[12] The use according to [11], wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C;
[13] The use according to any one of [10] to [12], wherein the polymer is used together with at least one selected from the group consisting of a metal salt compound and an amino acid;
[14] The use according to [13], wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof;
[15] The use according to [13] or [14], wherein the amino acid is at least one selected from the group consisting of α-alanine, arginine, asparagine, aspartic acid, glutamic acid, glycine, glycylglycine, methionine, phenylalanine, proline, serine, threonine, valine, and tricine;
[16] A polymer having 2-methacryloyloxyethyl phosphorylcholine as a constitutional unit to be used in shortening of blood coagulation time of a blood specimen deficient in coagulation factor XII in activated partial thromboplastin time measurement;
[17] The polymer according to [16], being a copolymer of 2-methacryloyloxyethyl phosphorylcholine and another monomer having a hydrophobic group, an anionic group, or a cationic group;
[18] The polymer according to [17], wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C;
[19] The polymer according to any one of [16] to [18], being used in a combination with at least one selected from the group consisting of a metal salt compound and an amino acid;
[20] The polymer according to [19], wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof;
[21] The polymer according to [19] or [20], wherein the amino acid is at least one selected from the group consisting of α-alanine, arginine, asparagine, aspartic acid, glutamic acid, glycine, glycylglycine, methionine, phenylalanine, proline, serine, threonine, valine, and tricine;
[22] Use of a polymer having 2-methacryloyloxyethyl phosphorylcholine as a constitutional unit in manufacturing of a blood coagulation time shortening agent for a blood specimen deficient in coagulation factor XII in activated partial thromboplastin time measurement;
[23] The use according to [22], wherein the polymer is a copolymer of 2-methacryloyloxyethyl phosphorylcholine and another monomer having a hydrophobic group, an anionic group, or a cationic group;
[24] The use according to [23], wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C;
[25] The use according to any one of [22] to [24], wherein the polymer is used together with at least one selected from the group consisting of a metal salt compound and an amino acid;
[26] The use according to [25], wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof; and
[27] The use according to [25] or [26], wherein the amino acid is at least one selected from the group consisting of α-alanine, arginine, asparagine, aspartic acid, glutamic acid, glycine, glycylglycine, methionine, phenylalanine, proline, serine, threonine, valine, and tricine.

### Advantageous Effects of Invention

According to the present invention, it is possible to shorten the blood coagulation times of FXII-deficient specimens in APTT measurement and thereby to precisely detect these blood specimens based on the blood coagulation times.

### Description of Embodiments

The present invention provides a blood coagulation time shortening agent (hereinafter, also referred to as "coagulation time shortening agent of the present invention") for FXII-deficient specimens in APTT measurement. The present invention also provides an APTT measurement reagent containing the coagulation time shortening agent. Furthermore, the present invention provides an APTT measurement method and a method for shortening the blood coagulation time of an FXII-deficient specimen in APTT measurement, using the coagulation time shortening agent.

The "FXII-deficient specimen" in this specification refers to a blood specimen that is deficient in coagulation factor XII (FXII), and examples thereof include a blood specimen collected from a patient deficient in FXII and commercially available FXII-deficient plasma.

Hereinafter, in this specification, the "blood specimen" and "blood coagulation time" may be simply referred to as "specimen" and "coagulation time", respectively. Accordingly, hereinafter, in this specification, "blood coagulation time shortening" and "blood coagulation time shortening agent" are also referred to as "coagulation time shortening" and "coagulation time shortening agent", respectively.

In APTT measurement, a sample solution is prepared by mixing a specimen and an APTT measurement reagent, and the blood coagulation reaction of the sample solution is measured. As the specimen, plasma of a subject is preferably used. To the specimen, an anticoagulant that is usually used in a coagulation test may be added. For example, blood is collected using a blood collection tube containing sodium citrate and is then centrifugated to obtain plasma. The APTT measurement reagent is generally composed of a first reagent including an activator and a second reagent including a coagulation initiator. A specimen is diluted with a diluent, as needed, before being mixed with an APTT measurement reagent. The first reagent is added to the specimen, incubation is performed for a predetermined time, and the second reagent is then added thereto to start the coagulation reaction. The coagulation time of the sample solution after the addition of the second reagent is measured.

The present invention provides a blood coagulation time shortening agent for FXII-deficient specimens in APTT measurement. In one embodiment, the coagulation time shortening agent of the present invention contains, as an active ingredient, a polymer having 2-methacryloyloxyethyl phosphorylcholine as a constitutional unit for shortening the blood coagulation time of an FXII-deficient specimen.

The polymer having 2-methacryloyloxyethyl phosphorylcholine as a constitutional unit (hereinafter, may be simply referred to as a polymer) is not particularly limited as long as the constitutional monomer has 2-methacryloyloxyethyl phosphorylcholine. The polymer having 2-methacryloyloxyethyl phosphorylcholine as a constitutional unit may be a 2-methacryloyloxyethyl phosphorylcholine homopolymer or may be a copolymer of 2-methacryloyloxyethyl phosphorylcholine and another monomer. The another monomer can include a hydrophobic group, an anionic group, or a cationic group. Alternatively, the another monomer may have both a hydrophobic group and a hydrophilic group. Preferably, the another monomer is a monomer in which a hydrophobic group, an anionic group, or a cationic group is bonded to a 2-methacryloyloxy group, and the copolymer has a methacryloyl group on the main chain and has phosphorylcholine and a hydrophobic group, an anionic group, or cationic group on the side chain.

The polymer having 2-methacryloyloxyethyl phosphorylcholine as a constitutional unit may be produced in accordance with a known synthesis method as shown in, for example, JP-A-2004-189678. The 2-methacryloyloxyethyl phosphorylcholine monomer is commercially available, for example, from NOF Corporation, and those skilled in the art can produce a polymer having required properties by polymerizing the monomer with an arbitrary monomer. As the polymer, a commercially available product, for example, a LIPIDURE (R) series that is available from NOF Corporation, can be used. The polymer is preferably a LIPIDURE (R)-BL series that is commercially available as a diagnostics field product, but is not limited thereto. The LIPIDURE-BL series is a group of copolymers in which various monomers are copolymerized with a 2-methacryloyloxyethyl phosphorylcholine monomer, and examples thereof include a LIPIDURE (R)-BL200 series that is a copolymer with a monomer having a hydrophobic group, a LIPIDURE (R)-BL400 series that is a copolymer with a monomer having an anionic group, a LIPIDURE (R)-BL500 series that is a copolymer with a monomer having a cationic group, and LIPIDURE (R)-BL800 series, LIPIDURE (R)-BL1000 series, LIPIDURE (R)-BL1100 series, LIPIDURE (R)-BL1200 series, and LIPIDURE (R)-BL1300 series that are copolymers with monomers having hydrophobic groups. More specific examples include LIPIDURE (R)-BL403, BL405, BL503, BL504, BL802, BL1002, BL1003, BL1103, BL1201, BL1301, and BL205.

The weight-average molecular weight of the polymer having 2-methacryloyloxyethyl phosphorylcholine as a constitutional unit is not particularly limited as long as desired performance can be obtained. The lower limit of the weight-average molecular weight of the polymer is, for example, 1,000, preferably 2,000, more preferably 5,000, and further more preferably 10,000. The upper limit of the weight-average molecular weight is, for example, 2,000,000, preferably 1,000,000, more preferably 700,000, further more preferably 100,000, and further more preferably 70,000. The weight-average molecular weight can be arbitrarily set in the range from any of the above-mentioned lower limits to any of the upper limits. The weight-average molecular weight of a polymer can be measured by a usual method such as gel permeation chromatography. When the polymer is a copolymer, the constitutional ratio of 2-methacryloyloxyethyl phosphorylcholine and another monomer in the copolymer varies depending on the structure and so on of the monomers to be used, but, generally, the content of 2-methacryloyloxyethyl phosphorylcholine in the copolymer is preferably 5 mol% or more. The weight-average molecular weight and monomer constitutional ratio of the polymer can be selected by those skilled in the art depending on the purpose using the above-described Lipidure (R)-BL series as a reference.

A polymer including 2-methacryloyloxyethyl phosphorylcholine as a constitutional unit preferably has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C. The surface tension and kinematic viscosity of the polymer can be selected by those skilled in the art depending on the purpose using the above-described Lipidure (R)-BL series as a reference.

The above-described polymers having 2-methacryloyloxyethyl phosphorylcholine as a constitutional unit may be used either alone or in combination of two or more.

The polymer is used for shortening the coagulation time of an FXII-deficient specimen in APTT measurement. The total amount of the polymer to be used in APTT measurement is not particularly limited as long as it is an effective amount for shortening the coagulation time of an FXII-deficient specimen in the APTT measurement. For example, the total amount of the polymer to be used in APTT measurement is, as the concentration of the polymer in the sample solution for the APTT measurement, preferably from 0.0005 to 0.2 mass%, more preferably from 0.001 to 0.1 mass%, further more preferably from 0.0075 to 0.075 mass%, and further more preferably from 0.01 to 0.05 mass%.

In one embodiment, the coagulation time shortening agent of the present invention can contain at least one selected from the group consisting of a metal salt compound and an amino acid mentioned below as an active ingredient for shortening the blood coagulation time of an FXII-deficient specimen. These metal salt compound and amino acid may be used alone as an active ingredient of the coagulation time shortening agent or in combination with the polymer as an active ingredient of the coagulation time shortening agent.

Examples of the metal salt compound include yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof. Examples of the hydrates include yttrium chloride hexahydrate, iron(III) chloride hexahydrate, indium(III) chloride tetrahydrate, copper(II) chloride dihydrate, potassium aluminum sulfate dodecahydrate, ammonium iron(III) sulfate dodecahydrate, and copper sulfate pentahydrate. The metal salt compounds mentioned above may be used either alone or in combination of two or more.

The metal salt compound is used for shortening the coagulation time of an FXII-deficient specimen in APTT measurement. The total amount of the metal salt compound to be used in APTT measurement is not particularly limited as long as it is an effective amount for shortening the coagulation time of an FXII-deficient specimen in the APTT measurement. For example, the total amount of the metal salt compound to be used in APTT measurement is, as the concentration of the metal salt compound in the sample solution for the APTT measurement, preferably from 0.0001 to 0.15 mg/mL, more preferably from 0.001 to 0.05 mg/mL, further more preferably from 0.005 to 0.04 mg/mL, and further more preferably from 0.0075 to 0.0225 mg/mL.

Examples of the amino acid include α-alanine, arginine, asparagine, aspartic acid, glutamic acid, glycine, glycylglycine, methionine, phenylalanine, proline, serine, threonine, valine, and tricine. These amino acids may be used either alone or in combination of two or more.

The amino acid is used for shortening the coagulation time of an FXII-deficient specimen in APTT measurement. The total amount of the amino acid to be used in APTT measurement is not particularly limited as long as it is an effective amount for shortening the coagulation time of an FXII-deficient specimen in the APTT measurement. For example, the total amount of the amino acid to be used in APTT measurement is, as the concentration of the amino acid in the sample solution for the APTT measurement, preferably from 10 to 500 mM, more preferably from 20 to 300 mM, further more preferably from 25 to 140 mM, and further more preferably from 30 to 135 mM.

The coagulation time shortening agent of the present invention can further contain a buffer or a polysaccharide or inclusion compound as an active ingredient for regulating the coagulation time of an FXII-deficient specimen, in addition to the above-described polymer, metal salt compound, or amino acid. The blood coagulation time of a specimen can be appropriately regulated, for example, prolongated or shortened, by using the buffer or the polysaccharide or inclusion compound in addition to the coagulation time shortening agent of the present invention.

The buffer is, for example, at least one selected from the group consisting of PIPES, ACES, HEPES, TAPSO, POPSO, EPPS, and CHES. The total amount of the buffer to be used in APTT measurement is, as the concentration in the sample solution, preferably from 0.1 to 1000 mM, more preferably from 0.5 to 500 mM, further more preferably from 1 to 200 mM, and further more preferably from 10 to 100 mM.

The polysaccharide is, for example, at least one polysaccharide selected from the group consisting of dextran, cyclodextrin, a sulfated polysaccharide, and salts thereof. Examples of the sulfated polysaccharide include dextran sulfate and cyclodextrin sulfate. Examples of the salt of sulfated polysaccharide include dextran sodium sulfate and cyclodextrin sodium sulfate. Examples of the inclusion compound include cyclodextrin and its modification and a calixarene derivative. Examples of the cyclodextrin and its modification include α-cyclodextrin sulfate, β-cyclodextrin sulfate, γ-cyclodextrin sulfate, and salts thereof. Examples of the salt include sodium salts. Examples of the calixarene derivative include calixarene (6) hemisulfate and calixarene (8) hemisulfate. The polysaccharides or inclusion compounds mentioned above may be used either alone or in combination of two or more. The total amount of the polysaccharide or inclusion compound to be used in APTT measurement is, as the concentration in the sample solution, preferably from 0.0001 to 0.15 mg/mL, more preferably from 0.001 to 0.05 mg/mL, and further more preferably from 0.001 to 0.01 mg/mL.

The buffers and polysaccharides or inclusion compounds mentioned above may be used either alone or in combination of two or more.

The coagulation time shortening agent of the present invention is used for shortening the coagulation time of an FXII-deficient specimen in APTT measurement. The coagulation time shortening agent of the present invention may be added alone to the specimen, diluent, or sample solution for APTT measurement, or an APTT measurement reagent containing the coagulation time shortening agent of the present invention may be added to the specimen, diluent, or sample solution.

Accordingly, the present invention provides an APTT measurement reagent containing the coagulation time shortening agent of the present invention. In one embodiment, the APTT measurement reagent of the present invention contains the polymer having 2-methacryloyloxyethyl phosphorylcholine as a constitutional unit and at least one selected from the group consisting of a metal salt compound and an amino acid. Preferably, the APTT measurement reagent of the present invention contains the polymer having 2-methacryloyloxyethyl phosphorylcholine as a constitutional unit. In one embodiment, the APTT measurement reagent of the present invention contains the polymer and at least one selected from the group consisting of the metal salt compound and amino acid. In one embodiment, the APTT measurement reagent of the present invention contains the polymer and the metal salt compound. In one embodiment, the APTT measurement reagent of the present invention can contain at least one selected from the group consisting of the buffer and polysaccharide or inclusion compound, in addition to the polymer and at least one selected from the group consisting of a metal salt compound and an amino acid. The APTT measurement reagent of the present invention can shorten the coagulation time of an FXII-deficient specimen.

Preferably, the APTT measurement reagent of the present invention is a reagent kit including a first reagent including an activator and a second reagent including a coagulation initiator. In the APTT measurement reagent, the coagulation time shortening agent of the present invention is preferably included in the first reagent, but may be included in the reagent kit separately from the first reagent and second reagent.

The content of the coagulation time shortening agent of the present invention in the APTT measurement reagent of the present invention may be controlled such that the concentrations of the active ingredients (e.g., the total amount of the polymer, metal salt compound, and amino acid) of the coagulation time shortening agent of the present invention included in a sample solution obtained by mixing the APTT measurement reagent and a specimen are within effective amount ranges for shortening the coagulation time of an FXII-deficient specimen in the APTT measurement. The contents of the buffer and polysaccharide or inclusion compound in the APTT measurement reagent are also the same.

The APTT measurement reagent provided by the present invention can contain, in addition to the coagulation time shortening agent of the present invention, various ingredients necessary for activation, coagulation, and so on of blood specimens. For example, in the APTT measurement reagent, the first reagent can contain a buffer, an activator, and a phospholipid.

As the buffer, a buffer having buffering capacity within a pH range of from 4 to 9, preferably from 6 to 8, can be appropriately selected and used. Examples of the buffer include the above-described buffers and an amino acid, citric acid, phosphoric acid, acetic acid, imidazole, barbital, and GTA. The amino acid that is an active ingredient of the above-described coagulation time shortening agent of the present invention can also be used as a buffer. These buffers can be used either alone or in combination of two or more. The amount of the buffer to be used is not particularly limited as long as the buffering capacity is exhibited, and the concentration in the reagent is preferably from 0.1 to 1000 mM, more preferably from 0.5 to 500 mM, further more preferably from 1 to 200 mM, and further more preferably from 10 to 100 mM.

Examples of the activator include ellagic acid, kaolin, celite, colloid silica, polyphenol compounds, silicic anhydride, and metal ions. Examples of the metal ion include Zn²⁺, Mn²⁺, Cu²⁺, Fe²⁺, and Al³⁺. The metal ion may be contained in the reagent in a salt form. The metal salt compound as the active ingredient of the above-described coagulation time shortening agent of the present invention can also be used as the activator. The above-mentioned activators may be used either alone or in combination of two or more. The amount of the activator to be used is not particularly limited, but the concentration in the reagent is preferably from 0.001 to 2 mg/mL, more preferably from 0.001 to 0.5 mg/mL, further more preferably from 0.001 to 0.1 mg/mL, and further more preferably from 0.005 to 0.05 mg/mL.

Examples of the phospholipid include natural and synthetic phospholipids. Examples of the natural phospholipid include phospholipids derived from natural substances, such as rabbit brain, bovine brain, human placenta, soybean, and egg yolk. Examples of the synthetic phospholipid include phosphatidylcholine (PC), phosphatidylethanolamine (PE), phosphatidylserine (PS), phosphatidylglycerol (PG), phosphatidic acid (PA), phosphatidylinositol (PI), lysophosphatidylcholine (LPC), sphingomyelin (SM), and cardiolipin. These phospholipids may be used either alone or in combination of two or more. For example, a mixture of multiple phospholipids can be used as liposome. The amount of the phospholipid to be used is not particularly limited, and the concentration in the reagent is preferably from 0.005 to 2 mM and more preferably from 0.02 to 0.5 mM. In the APTT measurement reagent, a phospholipid that is a substitute for platelet is contained as an ingredient necessary for the coagulation reaction. In the endogenous coagulation reaction, the phospholipid forms a complex of activated factor IX and factor VIII, and this complex activates factor X and lower common factors (factors V, II, and I) to lead to fibrin formation.

The second reagent of the APTT measurement reagent can contain an ingredient that initiates blood coagulation, such as a calcium ion. As the calcium ion, a water-soluble calcium compound, such as calcium chloride, calcium lactate, calcium gluconate, calcium glucuronate, and calcium tartrate, is used. These calcium compounds may be used either alone or in combination of two or more. The amount of the calcium compound to be used is not particularly limited, and the concentration in the reagent is preferably from 5 to 100 mM and more preferably from 10 to 50 mM.

The first reagent and the second reagent may further contain additives for improving the preservability or stability of the reagent. Examples of the additive include a preservative, an antioxidant, a dispersant, and a stabilizer. Examples of the preservative include ciprofloxacin, propionic acid, sodium benzoate, sodium azide, and a mixture of 2-methyl-1,2-thiazol-3(2H)-one and 5-chloro-2-methyl-1,2-thiazol-3(2H)-one (e.g., ProClin 300). Examples of the antioxidant include citric acid and butyl hydroxyanisole. Examples of the dispersant include phenol and collagen peptides. Examples of the stabilizer include polymer polysaccharides, such as polyethylene glycol, polyvinylpyrrolidone, dextran, and polysucrose (e.g., Ficoll); salts, such as sodium chloride; amino acids, and saccharides.

The coagulation time shortening agent and APTT measurement reagent of the present invention may be liquid or in their frozen forms or may be in dry forms. The reagent in a dry form is dissolved in water, a buffer solution, or the like just before using and is prepared into a liquid reagent. The concentration of each of the above ingredients represents the concentration in a liquid reagent.

A method for measuring blood coagulation reaction (APTT measurement) using the coagulation time shortening agent or APTT measurement reagent of the present invention is provided. The APTT measurement method by the present invention can shorten the coagulation time of an FXII-deficient specimen by using the coagulation time shortening agent or APTT measurement reagent of the present invention.

The APTT measurement method by the present invention may be carried out according to a usual method except that the coagulation time shortening agent or APTT measurement reagent of the present invention is used. In a preferable embodiment, a specimen diluted as needed is mixed with a first reagent including the coagulation time shortening agent of the present invention, and the resulting mixture solution is heated. The conditions of the heating are, for example, 30°C or more and 40°C or less and preferably 35°C or more and 39°C or less. Subsequently, a second reagent is added to the mixture solution to initiate the coagulation reaction. The coagulation reaction of the mixture solution after the addition of the second reagent (sample solution) is measured. In the measurement, for example, an optical method for measuring the amount of scattered light, transmittance, or absorbance of the sample solution or a mechanical method for measuring the viscosity of the sample solution can be used. The temperature of the sample solution during measurement is, for example, 30°C or more and 40°C or less and preferably 35°C or more and 39°C or less. The APTT measurement reagent is preferably added to a specimen in a concentration such that the coagulation time (APTT) of normal plasma is within a range of from 15 to 80 seconds, preferably from 15 to 60 seconds, and more preferably from 20 to 50 seconds.

Typically, the reaction initiation time point of the coagulation reaction may be defined as the time point at which the coagulation reaction is initiated by mixing a specimen with the second reagent. Alternatively, another timing may be defied as the reaction initiation time point. The time for continuing the measurement of the coagulation reaction may be, for example, from several tens seconds to about 7 minutes after the time point at which the specimen and the second reagent are mixed. This measurement time may be an arbitrarily determined fixed value, but may continue until the time point at which termination of the coagulation reaction of each specimen is detected. During the measurement period of time, measurement of the coagulation reaction progress (e.g., measurement of the amount of scattered light) may be repeated at predetermined intervals. For example, the measurement may be performed at 0.1 second intervals. The coagulation time (APTT) can be measured from the measured coagulation reaction according to a known method.

A series of steps in the APTT measurement method of the present invention can be performed using an automated analyzer. Alternatively, a part of steps may be manually performed. For example, specimens are prepared by a human, and subsequent steps can be performed by an automated analyzer.

### Examples

The present invention will now be described in further detail with reference to Examples, but the present invention is not limited to these Examples.

### Test 1

### 1) Specimen

The following specimens were used:
FXII-deficient specimens (specimens derived from FXII-deficient patients, N = 6).

### 2) APTT measurement reagent

### 2-1) Basic prescription

### First reagent (R1)

| Ingredient | Concentration |
|---|---|
| Ellagic acid | 0.01 mg/mL |
| Copper sulfate pentahydrate | 0.015 mg/mL |
| HEPES (pH 7.3) | 50 mM |
| Glycine | 133 mM |
| Liposome^{*1} | 0.15 mM |
| 3Na citrate | 0.1 mM |
| ProClin 300 | 0.05 mass% |

### Second reagent (R2)

| Ingredient | Concentration |
|---|---|
| Calcium chloride | 20 mM |
| Sodium azide | 0.05 mass% |

| | |
|---|---|
| *1) prepared according to the method described in WO 2003/015753. | |

### 2-2) Test reagent

Test reagents 1-1 to 1-22: a polymer having 2-methacryloyloxyethyl phosphorylcholine as a constitutional unit (LIPIDURE (R)-BL series; BL403, BL405, BL503, BL504, BL802, BL1002, BL1003, BL1103, BL1201, or BL1301) was added to R1 of the basic prescription under the conditions shown in Table 1.

Test reagents 2-1 to 2-10: yttrium chloride hexahydrate, manganese(II) chloride tetrahydrate, iron(III) chloride hexahydrate, aluminum chloride (anhydrate), indium (III) chloride tetrahydrate, copper(II) chloride dihydrate, or ammonium iron(III) sulfate dodecahydrate was added to R1 of the basic prescription instead of 0.0 15 mg/mL copper sulfate pentahydrate under the conditions shown in Table 2, or copper sulfate pentahydrate was used in a concentration shown in Table 2. As a control, R1 including zinc chloride instead of copper sulfate pentahydrate was prepared.

Test reagents 3-1 to 3-14: an amino acid was added to R1 of the basic prescription instead of glycine under conditions shown in Table 3. As a control, R1 including lysine instead of glycine was prepared.

### 2-3) Preparation of R1 and R2

Each test reagent and R1 and R2 as a control were prepared by mixing the above-mentioned ingredients at prescribed concentrations. Mixing of R1 was carried out under ice cooling.

### 3) Coagulation time measurement

A specimen (50 µL) was heated at 37°C for 45 seconds in a cuvette, and a first reagent (R1, 50 µL) was then added thereto, followed by heating for further 171 seconds. A second reagent (R2, 50 µL) was added to the heated mixture to start the coagulation reaction. The reaction was performed at 37°C. The R2-containing specimen (sample solution) was irradiated with light having a wavelength of 660 nm, and the optical variation (the amount of change in the scattered light intensity) due to the coagulation reaction was measured. From the measured coagulation reaction, the coagulation time was determined. The coagulation time measurement was performed using a blood coagulation automated analyzer CP3000 (manufactured by Sekisui Medical Co., Ltd.). The variation rate (%) of the coagulation time (APTT) with the test reagent with respect to the coagulation time (APTT) with the control (basic prescription), [variation rate (%) = (APTT with test reagent)/APTT with control × 100], was determined. A variation rate less than 100% represents that the coagulation time was shortened.

The results (average of the variation rates; N = 6) are shown in Tables 1 to 3. The coagulation times of FXII-deficient specimens are shortened compared to controls by the test reagents.

**[Table 1]**

| Test reagent | Concentration (mass%) | Polymer | | Variation rate (%) |
|---|---|---|---|---|
| | | Product name | Property of side chain | |
| Control 1 | 0 | - | - | 100 |
| 1-1 | 0.01 | Lipidure-BL403 | Anionic side chain | 84 |
| 1-2 | 0.03 | | | 81 |
| 1-3 | 0.05 | | | 71 |
| 1-4 | 0.01 | Lipidure-BL405 | Anionic side chain | 85 |
| 1-5 | 0.03 | | | 72 |
| 1-6 | 0.05 | | | 72 |
| 1-7 | 0.01 | Lipidure-BL503 | Cationic side chain | 77 |
| 1-8 | 0.01 | Lipidure-BL504 | Cationic side chain | 75 |
| 1-9 | 0.01 | Lipidure-BL802 | Hydrophobic side chai n | 83 |
| 1-10 | 0.03 | | | 90 |
| 1-11 | 0.05 | | | 94 |
| 1-12 | 0.01 | Lipidure-BL1002 | Hydrophobic side chai n | 85 |
| 1-13 | 0.03 | | | 67 |
| 1-14 | 0.05 | | | 66 |
| 1-15 | 0.01 | Lipidure-BL1003 | Hydrophobic side chai n | 89 |
| 1-16 | 0.03 | | | 85 |
| 1-17 | 0.05 | | | 85 |
| 1-18 | 0.01 | Lipidure-BL1103 | Hydrophobic side chai n | 94 |
| 1-19 | 0.03 | | | 93 |
| 1-20 | 0.05 | | | 95 |
| 1-21 | 0.01 | Lipidure-BL1201 | Hydrophobic side chai n | 78 |
| 1-22 | 0.01 | Lipidure-BL1301 | Hydrophobic side chai n | 71 |

**[Table 2]**

| Test reagent | Concentration (mg/mL) | Metal salt compound | Variation rate (%) |
|---|---|---|---|
| Control 2 | 0.015 | Zinc chloride | 100 |
| 2-1 | 0.015 | Yttrium chloride hexahydrate | 69 |
| 2-2 | 0.015 | Manganese(II) chloride tetrahydrate | 71 |
| 2-3 | 0.015 | Iron(III) chloride hexahydrate | 88 |
| 2-4 | 0.015 | Aluminum chloride (anhydrate) | 33 |
| 2-5 | 0.015 | Indium (III) chloride tetrahydrate | 32 |
| 2-6 | 0.015 | Copper(II) chloride dihydrate | 80 |
| 2-7 | 0.015 | Ammonium iron(III) sulfate dodecahydrate | 29 |
| 2-8 | 0.0075 | Copper sulfate pentahydrate | 95 |
| 2-9 | 0.015 | | 82 |
| 2-10 | 0.0225 | | 81 |

**[Table 3]**

| Test reagent | Concentration (mM) | Amino acid | Variation rate (%) |
|---|---|---|---|
| Control 3 | 133 | Lysine | 100 |
| 3-1 | 133 | α-Alanine | 47 |
| 3-2 | 133 | Arginine | 47 |
| 3-3 | 30 | Asparagine | 48 |
| 3-4 | 133 | Aspartic acid | 51 |
| 3-5 | 133 | Glutamic acid | 52 |
| 3-6 | 133 | Glycine | 44 |
| 3-7 | 133 | Glycylglycine | 47 |
| 3-8 | 40 | Methionine | 54 |
| 3-9 | 36 | Phenylalanine | 49 |
| 3-10 | 133 | Proline | 52 |
| 3-11 | 133 | Serine | 49 |
| 3-12 | 105 | Threonine | 50 |
| 3-13 | 81 | Valine | 48 |
| 3-14 | 133 | Tricine | 51 |

### Test 2

### 1) Specimen

The following specimens were used:
FXII-deficient specimens (specimens derived from FXII-deficient patients, N = 3).

### 2) APTT measurement reagent

### 2-1) Basic prescription

The prescription was the same as that in the test 1 except that copper sulfate pentahydrate in the first reagent (R1) was changed to zinc chloride.

### 2-2) Test reagent

Test reagent 4-1: LIPIDURE (R)-BL205 was added to R1 of the basic prescription under the conditions shown in Table 4. In the control, LIPIDURE (R) was not added.

Test reagents 5-1 to 5-3: zinc chloride in R1 of the basic prescription was changed to the metal salt compound shown in Table 5, and LIPIDURE (R)-BL205 was further added. As the control, the test reagent 4-1 was used.

### 2-3) Reagent preparation and coagulation time measurement

R1 and R2 were prepared by the same procedure as in the test 1, the coagulation time (APTT) was measured, and the variation rate (%) of APTT with the test reagent with respect to the control was determined.

The results (average of variation rates, N = 3) are shown in Tables 4 and 5. The coagulation times of FXII-deficient specimens were shortened by using a combination of a copolymer (LIPIDURE (R)) and zinc chloride compared to using zinc chloride alone with respect to the control. When manganese(II) chloride tetrahydrate, yttrium chloride (hexahydrate), or indium(III) chloride tetrahydrate was used instead of zinc chloride in combination with the polymer, the coagulation time was further shortened.

**[Table 4]**

| Test reagent | Control 4 | 4-1 |
|---|---|---|
| Lipidure^{™}-BL205 (mass%) | - | 0.01 |
| Zinc chloride (mg/mL) | 0.015 | 0.015 |
| APTT Variation rate (%) vs. Control (Control APTT: 248.7 sec) | 100 | 91 |

**[Table 5]**

| Test reagent | | | Control 5 (reagent 4-1) | 5-1 | 5-2 | 5-3 |
|---|---|---|---|---|---|---|
| | Lipidure^{™}-BL205 (mass%) | | 0.01 | 0.01 | 0.01 | 0.01 |
| | Metal salt compound (mg/mL) | | | | | |
| | | Zinc chloride | 0.015 | - | - | - |
| | | Manganese(II) chloride tetrahydrate | - | 0.015 | - | - |
| | | Yttrium chloride hexahydrate | - | - | 0.015 | - |
| | | Indium (III) chloride tetrahydrate | - | - | - | 0.015 |
| APTT Variation rate (%) vs. Control | | | 100 | 55 | 55 | 51 |

## Claims

1. A blood coagulation time shortening agent for a blood specimen deficient in coagulation factor XII, in activated partial thromboplastin time measurement, comprising, as an active ingredient, a polymer having 2-methacryloyloxyethyl phosphorylcholine as a constitutional unit.

2. The shortening agent according to Claim 1, wherein the polymer is a copolymer of 2-methacryloyloxyethyl phosphorylcholine and another monomer having a hydrophobic group, an anionic group, or a cationic group.

3. The shortening agent according to Claim 2, wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C.

4. The shortening agent according to Claim 1, further comprising at least one selected from the group consisting of a metal salt compound and an amino acid as an active ingredient.

5. The shortening agent according to Claim 4, wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof.

6. The shortening agent according to Claim 4, wherein the amino acid is at least one selected from the group consisting of α-alanine, arginine, asparagine, aspartic acid, glutamic acid, glycine, glycylglycine, methionine, phenylalanine, proline, serine, threonine, valine, and tricine.

7. An activated partial thromboplastin time measurement reagent comprising the blood coagulation time shortening agent according to any one of Claims 1 to 6.

8. An activated partial thromboplastin time measurement method comprising measuring a blood coagulation time of a sample solution containing a blood specimen and the blood coagulation time shortening agent according to any one of Claims 1 to 6.

9. A method for shortening blood coagulation time of a blood specimen deficient in coagulation factor XII in activated partial thromboplastin time measurement, comprising measuring blood coagulation time of a sample solution containing the blood specimen and the blood coagulation time shortening agent according to any one of Claims 1 to 6.

10. Use of a polymer having 2-methacryloyloxyethyl phosphorylcholine as a constitutional unit for shortening blood coagulation time of a blood specimen deficient in coagulation factor XII in activated partial thromboplastin time measurement.

11. The use according to Claim 10, wherein the polymer is a copolymer of 2-methacryloyloxyethyl phosphorylcholine and another monomer having a hydrophobic group, an anionic group, or a cationic group.

12. The use according to Claim 11, wherein the copolymer has a surface tension of from 44 × 10⁻³ to 66 × 10⁻³ N/m in a 0.1 wt% aqueous solution at 25°C and a kinematic viscosity of from 0.5 to 6 m²/s in a 5 wt% aqueous solution at 25°C, or has a surface tension of from 70 × 10⁻³ to 75 × 10⁻³ N/m in a 0.1 wt% aqueous solution at 25°C and a kinematic viscosity of from 2 to 7.3 m²/s in a 5 wt% aqueous solution at 25°C.

13. The use according to Claim 10, wherein the polymer is used together with at least one selected from the group consisting of a metal salt compound and an amino acid.

14. The use according to Claim 13, wherein the metal salt compound is at least one selected from the group consisting of yttrium chloride, iron(III) chloride, aluminum chloride, indium(III) chloride, copper chloride, potassium aluminum sulfate, ammonium iron(III) sulfate, copper sulfate, zinc chloride, manganese chloride, and hydrates thereof.

15. The use according to Claim 13, wherein the amino acid is at least one selected from the group consisting of α-alanine, arginine, asparagine, aspartic acid, glutamic acid, glycine, glycylglycine, methionine, phenylalanine, proline, serine, threonine, valine, and tricine.
